## Europäisches Patentamt
## European Patent Office
## Office européen des brevets

(11) Publication number: **0 058 428**
**B1**

(12) **EUROPEAN PATENT SPECIFICATION**

(45) Date of publication of patent specification: 09.10.85

(51) Int. Cl.⁴: **G 01 N 33/543**

(21) Application number: 82101136.8

(22) Date of filing: 16.02.82

(54) **An enzyme immuno-assay for simultaneously measuring a plurality of samples and test vessel for carrying out this method.**

(30) Priority: 18.02.81 JP 21366/81
17.04.81 JP 57057/81

(43) Date of publication of application:
25.08.82 Bulletin 82/34

(45) Publication of the grant of the patent:
09.10.85 Bulletin 85/41

(84) Designated Contracting States:
BE CH DE FR GB IT LI NL SE

(56) References cited:
GB-A-2 034 466
GB-A-2 051 357
GB-A-2 070 416
US-A-3 356 462
US-A-4 016 043
US-A-4 125 640
US-E- 27 756
US-E- 29 169

(73) Proprietor: Eisai Co., Ltd.
6-10, Koishikawa 4-chome Bunkyo-ku
Tokyo 112 (JP)

(72) Inventor: Namba, Yuzaburo
2-72-3, Sayamagaoka
Tokorozawa-shi Saitama Prefecture (JP)
Inventor: Naraki, Tohru
80-299, Akutamisuwayama
Gifu-shi Gifu Prefecture (JP)
Inventor: Sawada, Takashi
2, Takehayamachi Kawashima-cho
Hashima-gun Gifu Prefecture (JP)
Inventor: Kitamura, Toyoshiro
3-1032, Matsudo
Matsudo-shi Chiba Prefecture (JP)
Inventor: Tohda, Minoru
2-23, Ryokuennaka
Kakamigahara-shi Gifu Prefecture (JP)
Inventor: Morimoto, Tomiaki
2-10-10, Fuse-Shimmachi Kashiwa-shi
Chiba Prefecture (JP)

(74) Representative: Hansen, Bernd, Dr.rer.nat. et al
Hoffmann, Eitle & Partner Patentanwälte
Arabellastrasse 4
D-8000 München 81 (DE)

Courier Press, Leamington Spa, England.

(50) References cited:
**CLINICA CHIMICA ACTA, vol. 48, 1973,
AMSTERDAM (NL), L. BELANGER et al.:
"Enzyme-linked immunoassay for alpha-
fetoprotein by competitive and sandwich
procedures", pages 15-18
Gernot Peter: "Lehrbuch der klinischen
Chemie", page 259
Sokolowski/Wood: "Radioimmunoassay in
Theorie und Praxis", pages 43,44,143**

# 0 058 428

**Description**

This invention relates to an enzyme immuno-assay for simultaneously measuring a plurality of samples and test vessel for carrying out this method.

The sandwich method of enzyme immuno-assay is an excellent method which has been lately developed as a microassay of substances, as disclosed in Japanese Patent Publication No. 47011/1977, Japanese Patent Application Laid-open Nos. 32114/1977 and 57316/1977, "Igaku no Ayumi (Progress of Medicine)", Vol. 102, No. 2, pages 57—65. This method is being employed mainly for measuring antigenic substances, such as α-fetoprotein, HBs antigens, hormones etc.

On measuring a substance using this sandwich method of enzyme immunoassay, while it is possible to separately conduct measurements on the respective specimens, it is more common to conduct measurements collectively on a plurality of specimens in which the same substance is to be detected. When measuring collectively, the plurality of specimens are successively contacted at room temperature with an insolubilized binding agent specific for the substance to be measured, after which they are reacted at a temperature ranging from room temperature to 45°C and subjected to the subsequent steps to complete the measurement. However, in this case, there is the disadvantage in that a great fluctuation occurs in the results. This fluctuation is due to the fact that, since the plurality of specimens is successively contacted with the insolubilized binding agent specific for the substance to be measured, there is a considerable time lag between the specimen contacted first and the specimen contacted last. Such a time lag is the greatest in the above step, although some lag in time is also inevitably produced in the subsequent steps. Therefore, the more the number of the specimens to be measured at one time, the greater the fluctuation.

The method of this invention relates to an improvement for eliminating the above-described disadvantage. More particularly, this invention is characterized by the employment of cooling means, resulting in that the fluctuation of results can be minimized.

Accordingly, this invention provides an enzyme immuno-assay for simultaneously measuring a plurality of samples comprising the steps of

(a) reacting the substance to be measured (specimens) with an insolubilized binding agent specific for the substance to be measured;

(b) separating the reaction mixture of step (a) into the solid phase and the liquid phase;

(c) reacting the solid phase with an enzyme-labelled binding agent specific for the substance to be measured;

(d) separating the reaction mixture of step (c) into the solid phase and the liquid phase;

(e) reacting the solid phase or the liquid phase with a substrate for the enzyme of step (c); and

(f) measuring the optical density of the reaction mixture,

characterized in that in step (a) the addition of the samples to the insolubilized binding agent takes place under cooling conditions at a temperature ranging from 0°C to 15°C and is followed by simultaneous incubation at a temperature ranging from room temperature to 45°C.

Subsequent to the above step a, the above-described Steps b—f are conducted in a conventional manner to measure the substance in the specimens.

The reaction of the above step a is effected either by reacting the insolubilized material with the specimen in a reactor (e.g. a test tube) which has been previously arranged, or by adding the specimen to the insolubilized material which also serves as a reactor itself, e.g., a microtiter plate or cup, (that is, the reagent specific for the substance to be measured is coated on or fixed to the inner wall of the reactor) to induce the reaction. In this reaction, in order to control the temperatures ranging from 0°C to 15°C and also ranging from room temperature to 45°C, there may be used ice, cold water or an electric cooling device such as a cooling plate, as the cooling means and hot water bath, an incubator or a hot plate as the heating means, for such temperature control.

The Step b may be conducted by removing the reacted solution and washing the solid phase with water or buffer.

The Step c may be conducted by reacting the solid phase with an enzyme-labelled binding agent specific for the substance to be measured, at a temperature ranging from room temperature to 45°C. Also in this step, on successively adding the enzyme-labelled binding agent to the solid phase, it is desirable to add it at a temperature ranging from 0°C to 15°C and conduct the reaction at a temperature ranging from room temperature to 45°C in order to minimize the fluctuation of results.

In the Step d, the reacted solution and the solid phase are separated from each other. Where the solid phase is used in the subsequent Step e, the separated solid phase is washed with buffer or water.

The Step e may be effected by successively adding a solution of a substrate for the enzyme used to either the solid phase or the liquid phase obtained in the Step d, so as to carry out the enzymatic reaction, followed by adding, after a certain time, a stopping solution for the enzymatic reaction to the final reaction mixture. The temperature for the above enzymatic reaction is adjusted to the optimum temperature for the enzyme used.

The Step f may be conducted by measuring the optical density of the chromogen on a spectrophotometer using the wavelength suitable for the quantitative assay thereof.

In the above measuring system, the optical density is measured beforehand using a set of known

3

# 0 058 428

amounts of the substance and the calibration curve is obtained by plotting their amounts versus their optical densities. Thereafter, the optical density of the sample being determined is measured using the same measuring system, whereby the concentration of the analyte to be measured in the specimen is determined from the above calibration curve.

In this invention, on cooling or heating, the temperature control is easily effected by using a metal holder with which the outer wall of the reactor is closely contacted. For instance, a plate made of a good thermoconductor such as aluminum, copper, iron, is provided with a predetermined number of holes so that reactors can be inserted thereinto in close contact therewith, or such a shape is made by casting, and the reactors are inserted into such holes, and the temperature is controlled by the above-described cooling or heating means. In addition to the above shape, there may be mentioned a shape which has cylindrical protrusions so as to insert the reactors into the cylinders, or a shape wherein the outer wall of the reactors is covered with a metal foil or a metal plate. These may be constructed in such shape that the reactors are installable or removable, or the reactors are unified.

By employing such devices, the cooling and heating may be effected in a short time, thus shortening the time required for measuring. Further, the difference in temperature between the specimens is reduced, and therefore the fluctuation of results can be further reduced.

The solid phase which will be specifically combined with the substance to be measured, for use in this invention, may be obtained by fixing another binding agent specific for the substance being determined to an insoluble carrier. Examples of the material for such a carrier include polystyrene, cellulose, agarose, glass, cross-linked dextran, silicone rubber, metal. And, as to its shape, a tube, microtiter plate, cup, powder, sphere, disc, plate, flake may serve. For example, where a polystyrene microtiter plate is employed as the carrier for adsorbing the antibody, the antibody is diluted appropriately with a buffer, the diluted solution is added to the well, followed by incubation, thereby the antibody is fixed to the inner wall of the plate and the insolubilized antibody is thus obtained.

As the enzyme to be used for the preparation of an enzyme-labelled binding agent specific for the substance to be measured, there may be mentioned the enzymes which are conveniently employed in enzyme immunoassay. For instance, illustrative enzymes include alkaline phosphatase, peroxidase, $\beta$-D-galactosidase, glucoamylase, glucose oxidase. And as method for labelling the enzyme, there may be employed the glutaraldehyde method, Nakane method, the maleimide method, the mixed acid anhydride method, the carbodiimide method. For example, the enzyme is added to the antibody, glutaraldehyde is added thereto to a concentration of 0.2—0.8%, and the reaction is effected at room temperature, thereby the enzyme-labelled antibody is obtained.

As substrate, there is used a substrate for the enzyme employed in the preparation of the enzyme-labelled product. Where the enzyme is alkaline phosphatase, the substrate used may be p-nitrophenyl, phosphate, $\beta$-glycerol phosphate, phenyl phosphate, $\beta$-naphthyl phosphate, phenolphthalein phosphate, etc.

The solution for stopping the enzyme reaction may be a known solution specific for each enzyme. In the case of alkaline phosphatase, 1 N sodium hydroxide is suitable. From the above it will be understood that the substance which may be measured by the method of this invention is a substance which has a combination partner which will be specifically combined therewith. Examples of such a substance are those substances belonging to an immunological binding pair having the relationship: antigen-antibody, and hapten-antibody. For example, there may be mentioned cancer related antigens such as $\alpha$-fetoprotein, CEA, BFP (NEA), aldolase; hepatitis related antigens such as HB antigens (HBs, HBc, and HBe), HA antigen, non-A, non-B hepatitis antigen, hormones such as insulin, HCG, immunoglobulin, albumin, $\alpha$-macroglobulin, as well as their antibodies, etc.

For practising this invention, there is used a test vessel for the control of temperature which can be very readily and rapidly adjusted to the same intended temperature all over the vessel.

The above vessel comprises a receiver made of a synthetic resin providing a plurality of concave portions and in close contact thereto a base frame consisting of a thin layer plate or a foil which comprises a metal as a predominant component, the metal being capable of closely contacting to the outer wall of the receiver.

Such vessel can be rapidly controlled to a predetermined temperature, resulting in that the temperature difference between the specimens is reduced, and the reaction for the respective vessels may simultaneously proceed whereby the fluctuation of the results can be reduced. In addition, the cooling and heating may be effected in a short time, thus shortening the time required for measuring.

According to a further embodiment of this invention, a receptacle having a plurality of concave portions is shaped with a synthetic resin such as polystyrene, polyvinyl chloride, polyethylene, epoxy resin, and the like. The receptacle functions both as a reactor and as a binding material which specifically binds the partner substance to be measured.

A base frame for intimately covering the outer wall of the receptacle is fabricated with a thin layer plate or foil which comprises as a predominant component a good thermo-conductive metal such as aluminium, copper, iron. The frame is then unified to the receptacle. The frame has a function for rapidly conducting, to the receptacle, a controlled reaction temperature through the cooling means and heating means.

The base frame is unified with the receptacle by evaporation, or thermal melting, or adhering by means of ultrasonic waves, or an adhesive.

4

For example, when the receptacle of this invention is placed on a cooling plate or an ice, the frame made of a good thermo-conductive metal is at once cooled, resulting in that the temperature is lowered, and the receptacle made of a synthetic resin closely attached to the frame is cooled. The thermal conductivity to the receptacle has a rapid cooling effect in both a direct method for directly cooling and an indirect method which comprises cooling, e.g., by inserting it into a thermostat.

The same effect is achieved in a heating procedure, of course.

Figure 1 shows a test vessel of this invention, wherein a plurality of concave portions are provided.

Figure 2 shows a conventional vessel made of synthetic resin, wherein a plurality of concave portions are provided, and the frame is not contacted.

Figure 3 shows the comparison of the data of the sample, when the test vessel and the conventional vessel are respectively used.

This invention is more particularly described by the following examples.

Example 1.
Measurement of α-fetoprotein
a) Antihuman α-fetoprotein antibody coated cups

To each polystyrene cup (6.5 mm in inner diameter and 10 mm in depth) was added 150 µl of a 0.05 M tris-hydrochloric acid buffer (pH 8.0) containing 10 µg/ml of a purified antihuman α-fetoprotein antibody (rabbit), and this was allowed to stand at 4°C overnight. The liquor portion was rmeoved from each cup, which was then washed with distilled water, whereby the immobilized antihuman α-fetoprotein antibody had been obtained.

b) Alkaline phosphatase-labelled antihuman α-fetoprotein antibody

To 1 ml of an alkaline phosphatase solution (containing 3 mg of protein and exhibiting 1000 units per mg of specific activity) was added 0.5 ml of a 0.05 M tris-hydrochloric acid buffer containing 3 mg of a purified antihuman α-fetoprotein antibody. To the resulting mixed solution was added a 25% aqueous glutaraldehyde solution to adjust the glutaraldehyde concentration to 0.2%. The mixture was allowed to stand at room temperature for 3 h. Thereafter, this was dialyzed against a 0.05 M tris-hydrochloric acid buffer (pH 8.0) overnight, to obtain an alkaline phosphatase-labelled antihuman α-fetoprotein antibody.

c) Enzyme immunoassay
Measuring system A

The cups obtained in the above a) were inserted into the respective holes on an aluminum plate (aluminum holder) having 96 such holes (8 holes across×12 holes lengthwise).

1) This aluminum holder was placed on ice cube, and under the cooling condition 100 µl portions of α-fetoprotein positive human serum (the α-fetoprotein concentration of 120 ng/ml and the temperature of the specimens of 19°C) was added to the respective cups No 1—48; 100 µl portions of the α-fetoprotein standard specimen (for preparing a calibration curve) to the respective cups Nr. 49—56; and 100 µl portions of the above α-fetoprotein positive human serum to the remaining respective cups Nr. 57—96, successively in the numerical order and using micropipettes.

2) The aluminum holder was dipped in warm water at 37°C, and the reaction was effected for 60 min.

3) The aluminum holder was taken out from the warm water and the respective internal liquors were successively removed by suction using an aspirator, after which each cup was washed with distilled water (i.e. by adding distilled water and removing it by suction) three times.

4) The aluminum holder was placed on ice cube. To the respective cups were respectively added 100 µl portions (4°C) of a 400-fold dilution of the alkali phosphatase-labelled antihuman α-fetoprotein antibody obtained in the above step b) with 50% rabbit serum.

5) The aluminum holder was dipped in warm water at 37°C, and the reaction was effected for 60 min.

6. Similarly as in the above step 3) the removal of the cup internal liquor and washing were conducted.

7) The aluminum holder was placed on ice cubes, and 100 µl portions (4°C) of an aqueous p-nitrophenyl phosphate solution (4 mg/ml) were successively added to the respective cups.

8) The aluminium holder was again dipped in warm water of 37°C, and the reaction was effected for 60 min.

9) The aluminum holder was taken out from the warm water and placed on ice cubes to cool for 2 min, after which 100 µl portions (4°C) of a 1 N sodium hydroxide were successively added to the respective cups.

10) Each cup internal liquor was diluted with distilled water 11 times, and the optical density (OD) of each liquor was measured with a spectrophotometer at 405 nm.

A calibration curve was prepared from the $OD_{405\ nm}$ of the cups Nr. 49—56, and the α-fetoprotein concentrations of Nr. 1—48 and Nr. 57—96 were obtained.

Measuring system B
in steps 3) and 6) of the Measuring System A, the aluminum holder taken out from the warm water was placed on ice cubes for 2 min to cool, and thereafter the remaining respective operations were carried out.

Measuring system C

In steps 4), 7) and 9) of the Measuring System A, the respective operations were carried out at room temperature (19°C), without cooling the aluminum holder with ice cubes.

Measuring system D

In steps 1), 4), 7) and 9) of the Measuring System A, the respective operations were carried out at room temperature (19°C), without cooling the aluminium holder with ice cubes.

The results of the measurements of α-fetoprotein by the Measuring Systems A—D are given in Table 1.

TABLE 1

| Specimen (cup Nr.) | Average value mg/ml | | | | Coefficient of (c.v.) % variation | | | |
|---|---|---|---|---|---|---|---|---|
| | A | B | C | D (control) | A | B | C | D (control) |
| 1~16 | 122.8 | 108.8 | 117.1 | 142.6 | 4.9 | 1.9 | 9.0 | 8.4 |
| 17~32 | 124.3 | 114.9 | 111.3 | 127.1 | 4.1 | 4.2 | 6.1 | 7.3 |
| 33~48 | 128.7 | 114.7 | 112.6 | 116.7 | 2.4 | 7.0 | 5.6 | 6.5 |
| 57~64 | 131.8 | 115.6 | 109.1 | 113.9 | 1.6 | 3.8 | 4.7 | 6.2 |
| 65~80 | 124.4 | 116.7 | 109.9 | 112.8 | 4.6 | 2.6 | 4.0 | 6.3 |
| 81~96 | 120.3 | 116.4 | 109.7 | 96.8 | 3.7 | 3.1 | 4.8 | 7.1 |
| Total specimens (Nr. 1—48, & 57—96) | 124.8 | 114.4 | 111.8 | 117.6 | 4.6 | 4.7 | 6.4 | 17.5 |

As shown in Table 1, in the Measuring System D (control) where no cooling operation was conducted, the fluctuation in measured values is very broad. In other words, the coefficient of variation for the total specimens ranges as broad as 17.5%. In addition, the average value for Nr. 1—16 amounts to 142.6 ng/ml, while the average value for Nr. 81—96 amounts to 96.8 ng/ml. That is, there is a disadvantage that the results are greatly different from one another depending on the order in the measuring operation.

On the contrary, in the Measuring System A, B and C where the cooling operation was conducted in accordance with this invention, there is achieved a characteristic advantage that the fluctuation of results is very narrow.

Example 2

Measurement of α-fetoprotein

Using the Measuring System A in Example 1, α-fetoprotein positive human serum (the α-fetoprotein concentration of 120 ng/ml and the temperature of the specimens of 4°C) was measured. As the control, the measurements were conducted using the Measuring System D in Example 1. The results are given in Table 2.

**0 058 428**

TABLE 2

| Specimen (cup Nr.) | Average value ng/ml | | Coefficient of variation (c.v.) % | |
|---|---|---|---|---|
| | This invention | Control | This invention | Control |
| 1—16 | 115.3 | 142.9 | 2.5 | 11.3 |
| 17—32 | 124.1 | 131.2 | 3.5 | 4.5 |
| 33—48 | 113.3 | 125.9 | 3.4 | 3.3 |
| 57—64 | 122.3 | 124.6 | 3.2 | 3.8 |
| 65—80 | 115.8 | 117.0 | 5.3 | 2.8 |
| 81—96 | 119.6 | 113.9 | 3.6 | 4.1 |
| Total specimens (Nr. 1—48 & 57—96) | 118.0 | 126.1 | 4.9 | 10.1 |

As shown in the above table, also when conducting the measurement of the specimens which have been previously cooled to 4°C, the fluctuation of results and coefficient of variation are smaller, when the cooling operation according to this invention is additionally conducted.

Example 3

Measurement of α-fetoprotein

The measurement of α-fetoprotein positive human serum (the α-fetoprotein concentration of 130 ng/ml and the temperature of the specimens of 28°C) was conducted according to the Measuring System A in Example 1. For assays of the specimens cups Nr. 1—96 were used. The assay of the standard specimen was conducted with separately prepared cups at the time between the assays of the cups Nr. 48 and 49. The room temperature was 28°C. Instead of cooling with ice cubes in steps 1), 4), 7) and 9) in the Measuring System A in Example 1, the cooling was effected using water of 3°C, 7°C, 10°C and 15°C, respectively. For the controls, the operations were conducted at room temperature (28°C) and with cooling using cold water of 20°C in steps 1), 4), 7) and 9). The results are given in Table 3.

TABLE 3

| Cooling temperature | Specimen Nr. 1 ng/ml | Specimen Nr. 96 ng/ml | Average value (Nr. 1—96) ng/ml | Coefficient of variation (c.v.) (Nr. 1—96) % |
|---|---|---|---|---|
| 28°C. (control) | 225 | 95 | 129 | 24.9 |
| 20°C. (control) | 171 | 111 | 127 | 15.8 |
| 15°C. | 150 | 116 | 128 | 11.5 |
| 10°C. | 137 | 127 | 132 | 8.7 |
| 7°C. | 138 | 129 | 128 | 7.2 |
| 3°C. | 141 | 140 | 131 | 6.0 |

As shown in Table 3, the lower the cooling temperature, the smaller the fluctuation of results and coefficient of variation.

Example 4

Measurement of human muscle type aldolase

a) Antihuman muscle type aldolase antibody coated cups

Using a purified antihuman muscle type aldolase antibody (chicken), procedures similar to those of

7

step a) of Example 1 were conducted to obtain cups to which the antihuman muscle type aldolase antibody had been combined.

b) Alkaline phosphatase-labelled antihuman α-fetoprotein antibody

Using the purified antihuman muscle type aldolase antibody (chicken), procedures similar to those of step b) of Example 1 were conducted to obtain an alkaline phosphatase-labelled antihuman α-fetoprotein antibody.

c) Enzyme immunoassay

Into the aluminum holder employed in Example 1 were inserted 54 cups obtained in the above step a). This aluminum holder was placed on ice cubes and under cooling condition 100 µl portions of human muscle type aldolase positive human serum (the human muscle type aldolase concentration of 250 ng/ml and the temperature of the specimens of 19°C) were added to the respective cups Nr. 1—24; 100 µl portions of the human muscle type aldolase standard specimen (for preparing a calibration curve) to the respective cups Nr. 25—32; and 100 µl portions of the above human muscle type aldolase positive human serum to the respective cups Nos. 33—56, successively in the numerical order. The aluminum holder was dipped in warm water at 37°C, and the reaction was effected for 60 min. The aluminum holder was taken out from the warm water, and each cup internal liquor was removed by suction using an aspirator, and washed with distilled water (i.e. adding distilled water and removing it by suction) three times. At room temperature (19°C), 100 µl portions (4°C) of a 400-fold dilution of the alkaline phosphatase-labelled antihuman muscle type aldolase antibody obtained in the above step b) with 50% rabbit serum were successively added to the respective cups. The aluminum holder was dipped in warm water at 37°C, and the reaction was effected for 60 min. The aluminum holder was then taken out from the warm water, and after removing each cup internal liquor by suction, the cup was washed with distilled water three times similarly as the above. At room temperature, 100 µl portions (4°C) of an aqueous p-nitrophenyl phosphate solution (4 mg/ml) were successively added to the respective cups. The aluminum holder was dipped in warm water at 37°C, and the reaction was effected for 60 min. The aluminum holder was taken out from the warm water, and 100 µl portions (4°C) of a 1 N sodium hydroxide were successively added to the respective cups. Each cup internal liquor was diluted 11 times with distilled water, and the optical density was measured on a spectrophotometer at 405 nm. A calibration curve was prepared, from which the concentration of the human muscle type aldolase in each specimen was determined. The results are given in the following table.

TABLE 4

| Specimen (cup Nr.) | Average value ng/ml | Coefficient of variation (c.v.) % |
|---|---|---|
| 1—16 | 260.4 | 5.7 |
| 17—24, 33—40 | 253.1 | 5.1 |
| 41—56 | 248.9 | 5.5 |
| Total specimen (Nr. 1—24 & 33—56) | 254.0 | 5.6 |

Example 5
Measurement of HBs antigen
a) Anti-HBsAg antibody coated cups

Using a purified anti-HBs antibody (rabbit), procedures similar to those of step a) of Example 1 were conducted to obtain cups to which the anti-HBs antibody has been combined.

b) Alkaline phosphatase-labelled anti-HBs antibody

Using the purified anti-HBsAg antibody (rabbit), procedures similar to those of step b) of Example 1 were conducted to obtain an alkaline phosphatase-labelled anti-HBsAg antibody.

c) Enzyme immunoassay

Into the aluminum holder employed in Example 1 were inserted 18 cups obtained in the above step a). This aluminum holder was placed on ice cubes, and under cooling condition 100 µl portions of HBs antigen positive human serum (the HBs antigen concentration of 210 ng/ml and the temperature of the specimens of 19°C) were added to the respective cups Nr. 1—6, 100 µl portions of the HBs antigen standard specimen (for preparing a calibration curve) to the respective cups Nr. 7—12, and 100 µl portions of the above HBs antigen positive human serum to the respective cups Nr. 13—18, successively in the numerical order. The aluminum holder was dipped in warm water at 37°C, and the reaction was effected for 60 min. The

8

aluminum holder was then taken out from the warm water, and the cup contents were removed successively by suction using an aspirator, and washed with distilled water (i.e. adding distilled water and removing it by suction) three times. At room temperature (19°C), 100 µl portions (4°C) of a 400-fold dilution of the alkaline phosphatase-labelled anti-HBsAg antibody obtained in the above step b) with 50% rabbit serum were successively added to the respective cups. Thereafter, similar procedures as those in Example 4 were conducted, to measure the HBs antigen in the specimens. The average value of the total specimens (Nr. 1—6 and 13—18) amounted to 211.1 ng/ml and the coefficient of variation (c.v.) amounted to 4.2%.

Example 6

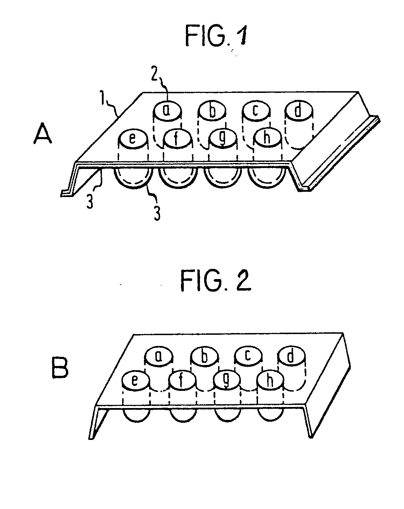

With reference to Figure 1, a receiver 1 made of synthetic resin is prepared by providing concave portions 2 such as eight cup-like concaves a, b, c, d, e, f, g and h, each having an inner diameter of 8 mm and a depth of 10 mm.

The frame 3 which was prepared by casting an aluminium plate is closely contacted to the outer wall of the receiver 1, in order to prepare a test vessel A. To the respective concaves were added 100 µl portions of α-fetoprotein positive human serum (the α-fetoprotein concentration of 120 ng/ml). The subsequent procedures are carried out in accordance with Example 1, Measuring System A. The optical density (OD) was measured on a spectrophotometer at 405 nm.

Conventional vessel B is used as shown in Figure 2, wherein the frame is not contacted.

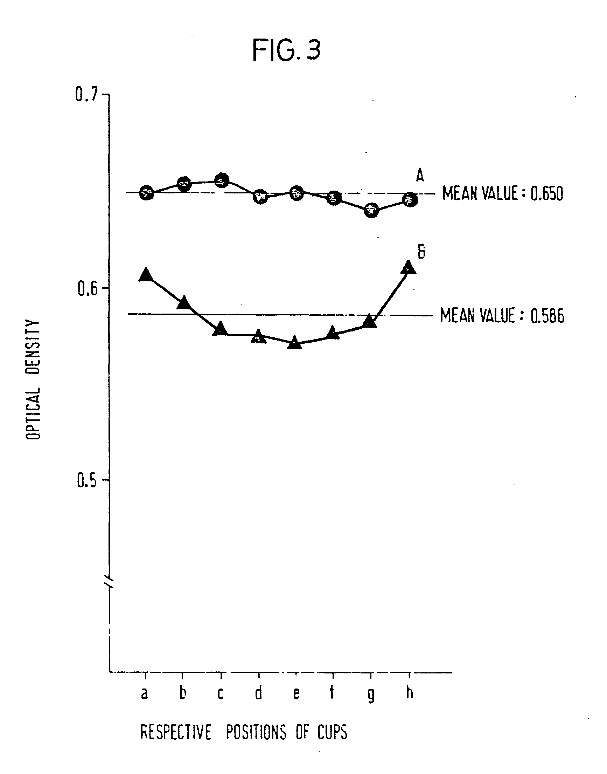

Figure 3 shows the data of the optical densities for the sample by the use of the test vessel A of this invention and the conventional vessel B. It is apparent that the data obtained with the test vessel A show less fluctuation than those obtained with the conventional vessel B. There is also obtained an advantageous merit that the mean values of the optical densities of the former are higher than those of the latter, (above 10%).

**Claims**

1. An enzyme immuno-assay for simultaneously measuring a plurality of samples comprising the steps of

(a) reacting the substance to be measured (specimens) with an insolubilized binding agent specific for the substance to be measured;

(b) separating the reaction mixture of step (a) into the solid phase and the liquid phase;

(c) reacting the solid phase with an enzyme-labelled binding agent specific for the substance to be measured;

(d) separating the reaction mixture of step (c) into the solid phase and the liquid phase;

(e) reacting the solid phase or the liquid phase with a substrate for the enzyme of step (c); and

(f) measuring the optical density of the reaction mixture,

characterized in that in step (a) the addition of the samples to the insolubilized binding agent takes place under cooling conditions at a temperature ranging from 0°C to 15°C and is followed by simultaneous incubation at a temperature ranging from room temperature to 45°C.

2. An immuno-assay according to claim 1, characterized in that the successive addition of the enzyme-labelled product to the solid phase in the step (c) is effected at a temperature ranging from 0°C to 15°C, and that the reaction is then conducted at a temperature ranging from room temperature to 45°C.

3. A test vessel for carrying out the enzyme immuno-assay according to claim 1 or 2, characterized by a receptacle made of a synthetic resin endowed with a plurality of concave portions, and in close contact thereto a base frame consisting of a thin layer plate or a foil, which comprises a metal as a predominant component, the metal being capable of closely contacting the outer wall of the receptacle.

**Patentansprüche**

1. Enzym-Immunoverfahren zur gleichzeitigen Messung einer Vielzahl von Proben, gekennzeichnet durch die folgenden Schritte:

(a) Umsetzung der zu messenden Substanz (Proben) mit einem insolubilisierten Bindungsagens, welches für die zu messende Substanz spezifisch ist;

(b) Trennung des Reaktionsgemisches von Schritt (a) in eine feste Phase und eine flüssige Phase;

(c) Umsetzung der festen Phase mit einem Enzym-markierten Bindungsagens, welches für die zu messende Substanz spezifisch ist;

(d) Trennung des Reaktionsgemisches von Schritt (c) in eine feste Phase und eine flüssige Phase;

(e) Umsetzung der festen Phase oder der flüssigen Phase mit einem Substrat für das Enzym von Schritt (c); und

(f) Bestimmung der Extinktion des Reaktionsgemisches, dadurch gekennzeichnet, daß in Schritt (a) die Zugabe der Proben zu dem insolubilisierten Bindungsagens in der Kälte bei einer Temperatur im Bereich von 0°C bis 15°C erfolgt und daraufhin die gleichzeitige Inkubation bei einer Temperatur im Bereich von Raumtemperatur bis 45°C erfolgt.

2. Immunoverfahren nach Anspruch 1, dadurch gekennzeichnet, daß die sukzessive Zugabe von Enzymmarkiertem Produkt zu der festen Phase in Schritt (c) bei einer Temperatur von 0°C bis 15°C durchgeführt wird und die Reaktion dann bei einer Temperatur im Bereich von Raumtemperatur bis 45°C erfolgt.

3. Testgefäß zur Durchführung des Enzym-Immunoverfahrens nach Anspruch 1 oder 2, dadurch gekennzeichnet, daß eine aus synthetischem Harz hergestellte Vorrichtung zur Aufnahme der Proben mit einer Vielzahl von Vertiefungen versehen ist und sich in engem Kontakt mit einem Basisrahmen befindet, der aus einer dünnschichtigen Platte oder einer Folie besteht, welche als Hauptbestandteil ein Metall umfaßt, wobei das Metall in der Lage ist, einen engen Kontakt mit der Außenwand der Vorrichtung zur Aufnahme der Proben herzustellen.

**Revendications**

1. Titrage immuno-enzymatique pour la mesure simultanée de plusieurs échantillons, comprenant les étapes qui consistent à:

(a) faire réagir la substance à mesurer (échantillons) avec un agent de liaison insolubilisé spécifique de la substance à mesurer;

(b) séparer le mélange réactionnel de l'étape (a) en la phase solide et la phase liquide;

(c) faire réagir la phase solide avec un agent de liaison marqué par une enzyme et spécifique de la substance à mesurer;

(d) séparer le mélange réactionnel de l'étape (c) en la phase solide et la phase liquide;

(e) faire réagir la phase solide ou la phase liquide avec un substrat convenant à l'enzyme de l'étape (c); et

(f) mesurer la densité optique du mélange réactionnel, caractérisé en ce que, dans l'étape (a), l'addition des échantillons à l'agent de liaison insolubilisé a lieu sous des conditions de refroidissement à une température se situant dans la plage de 0°C à 15°C et est suivie par une incubation simultanée à une température se situant dans la plage allant de la température ambiante à 45°C.

2. Titrage immunologique selon la revendication 1, caractérisé en ce que, dans l'étape (c), l'addition successive du produit marqué par une enzyme à la phase solide est effectuée à une température se situant dans la plage de 0°C à 15°C, et la réaction est ensuite conduite à une température se sitant dans la plage allant de la température ambiante à 45°C.

3. Récipient d'essai pour effectuer le titrage immuno-enzymatique selon la revendication 1 ou 2, caractérisé par un réceptacle constitué d'une résine synthétique pourvu de plusieurs parties concaves, et, en contact étroit avec celui-ci, un châssis de base constitué d'une plaque en couche mince ou d'une feuille, qui comprend un métal comme composant prédominant, le métal étant à même de se trouver en contact étroit avec la paroi externe du réceptable.

0 058 428

FIG. 1

A

FIG. 2

B

1

# FIG. 3